# FASCICULE DE BREVET EUROPEEN

(11) **EP 1 865 886 B1**
(45) Date de publication et mention de la délivrance du brevet: **23.03.2016**
(21) Numéro de dépôt: 06726146.1
(22) Date de dépôt: 24.03.2006
(51) Int. Cl.: A61F 2/24

(54) **NECESSAIRE DESTINE A ETRE IMPLANTE DANS UN CONDUIT DE CIRCULATION DU SANG, ET ENDOPROTHESE TUBULAIRE ASSOCIEE**
IN EIN BLUTGEFÄSS ZU IMPLANTIERENDES KIT UND VERWANDTE RÖHRENFÖRMIGE ENDOPROTHESEN
KIT DESIGNED TO BE IMPLANTED IN A BLOODSTREAM DUCT, AND RELATED TUBULAR ENDOPROSTHESIS

(30) Priorité: 05.04.2005 FR 0503374
(43) Date de publication de la demande: 19.12.2007
(73) Titulaire: Cormove, 60540 Bornel (FR)
(72) Inventeur: STYRC, Mikolaj, Witold, L-8190 Kopstal (LU); PEROUSE, Eric, F-75016 Paris (FR)
(74) Mandataire: Blot, Philippe Robert Emile
(86) Numéro de dépôt international: PCT/FR2006/000660
(87) Numéro de publication internationale: WO 2006/106205

(56) Documents cités:
- EP-A- 0 850 607
- WO-A-00/47139
- WO-A-01/56512
- WO-A-02/22054
- WO-A-91/17720
- FR-A- 2 847 800

## Description

La présente invention concerne un nécessaire, destiné à être implanté dans un conduit de circulation du sang, du type comprenant :
- une endoprothèse tubulaire présentant une surface intérieure délimitant un canal d'axe longitudinal ; et
- une valve prothétique destinée à être implantée dans le canal, la valve comprenant :
   - une armature porteuse présentant une surface extérieure destinée à être appliquée contre la surface intérieure de l'endoprothèse, l'armature étant déformable radialement depuis une position repliée de mise en place jusqu'à une position déployée d'implantation ;
   - un obturateur souple, lié à l'armature et déformable entre une position d'obstruction dans laquelle il est étendu transversalement et une position de libération dans laquelle il est contracté transversalement sous l'action du flux circulant dans le canal.

On connaît de EP-A-0 850 607 un nécessaire du type précité comprenant une endoprothèse tubulaire et une valve prothétique qui présente une armature porteuse déformable et un obturateur souple fixé sur l'armature.

On connaît également les nécessaires décrits par WO 00/47139 et par FR 2 847 800.

Un tel nécessaire est destiné à être implanté en remplacement d'une valve, dans un conduit de circulation du sang.

De telles valves sont par exemple présentes dans le coeur, entre les oreillettes et les ventricules, ou à la sortie du ventricule droit et du ventricule gauche. Ces valves assurent une circulation univoque du flux sanguin, évitant un reflux sanguin à l'issue de la contraction ventriculaire.

Pour effectuer un remplacement de valve, l'endoprothèse tubulaire prévue dans le nécessaire est implantée dans la partie du conduit dans lequel se trouve la valve malade. Puis, la valve prothétique dans son état replié est amenée dans le canal intérieur délimité par l'endoprothèse et est appliquée contre cette endoprothèse par gonflage d'un ballon.

Un tel dispositif ne donne pas entière satisfaction. En effet, le positionnement relatif de la valve prothétique par rapport à l'endoprothèse est approximatif, et la fixation de la valve dans l'endoprothèse est peu robuste.

Un but de l'invention est donc de fournir un nécessaire du type précité, qui permette l'implantation successive d'une endoprothèse tubulaire dans un conduit de circulation du sang humain, puis d'une valve prothétique dans l'endoprothèse, de sorte que le positionnement de la valve prothétique dans l'endoprothèse soit aisé et robuste.

A cet effet, l'invention a pour objet un nécessaire selon la revendication 1.

Le nécessaire selon l'invention peut comprendre une ou plusieurs des caractéristiques suivantes, prise(s) isolément ou suivant toutes combinaisons techniquement possibles :
- les deux tronçons de section transversale variable sont délimités par deux étranglements annulaires qui font saillie dans le canal ; et
- les tronçons de section transversale variable sont radio-opaques.

L'invention a également pour objet une endoprothèse tubulaire selon la revendication 4.

L'invention a enfin pour objet une valve prothétique selon la revendication 5.

L'invention sera mieux comprise à la lecture de la description qui va suivre, donnée uniquement à titre d'exemple et faite en se référant aux dessins annexés, sur lesquels :
- la Figure 1 est une vue de dessus d'un premier nécessaire selon l'invention ;
- la Figure 2 est une vue en coupe transversale du premier nécessaire selon l'invention implanté dans un conduit de circulation du sang ;
- la Figure 3 est une vue analogue à la Figure 2 d'un deuxième nécessaire selon l'invention ;
- la Figure 4 est une vue analogue à la Figure 2 d'un troisième nécessaire selon l'invention ;
- la Figure 5 est une vue en perspective d'une valve d'un quatrième nécessaire selon l'invention ; et
- la Figure 6 une vue en coupe suivant un plan transversal du quatrième nécessaire selon l'invention implanté dans un conduit de circulation du sang.

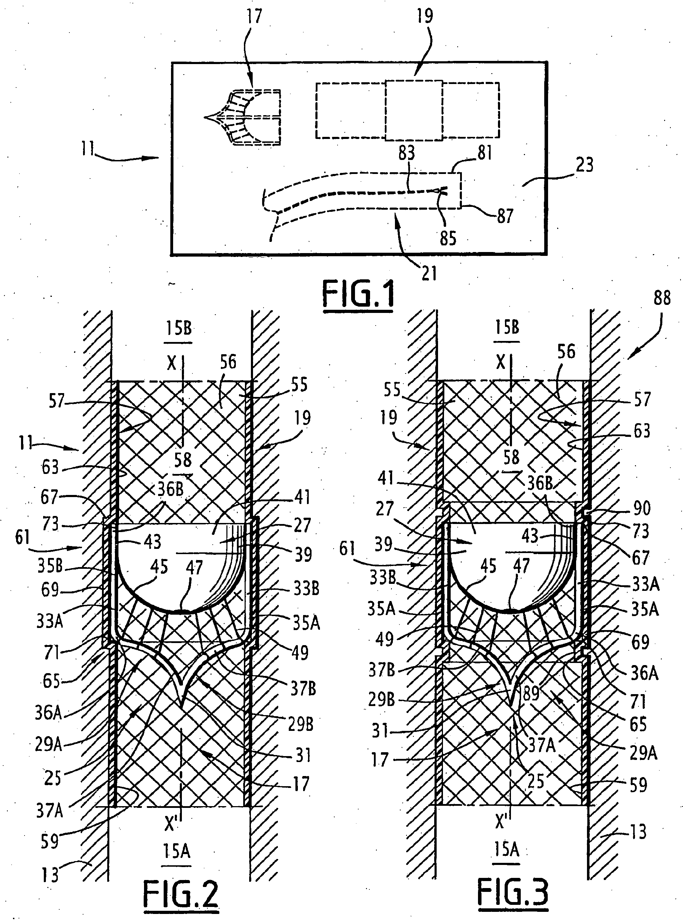

Un premier nécessaire selon l'invention est représenté sur les Figures 1 et 2.

La Figure 1 représente le nécessaire 11 avant son implantation dans un conduit de circulation du sang, alors que la Figure 2 représente le nécessaire 11 implanté dans un conduit 13 de circulation du sang. Le conduit 13 est par exemple une artère pulmonaire reliée à son extrémité proximale 15A à la sortie du ventricule droit du coeur, notamment d'un être humain, et à son extrémité distale 15B au poumon.

Comme illustré par la Figure 1, le nécessaire 11 comprend une valve prothétique 17, une endoprothèse 19 destinée à recevoir la valve prothétique 17 et des moyens 21 d'implantation et d'extraction de la valve prothétique 17 dans l'endoprothèse 19.

Le nécessaire 11 est par exemple conservé dans un emballage 23.

Comme illustré par la Figure 2, la valve prothétique interchangeable 17 comporte une armature porteuse 25 et un obturateur déformable 27 supporté par l'armature 25 et solidaire de celle-ci. La valve 17 est généralement d'axe longitudinal X-X'.

L'armature porteuse 25 comporte des moyens intégrés pour sa compression centripète. Plus précisément, l'armature 25 est formée d'au moins deux branches 29A, 29B et notamment trois reliées l'une à l'autre à une première extrémité 31 pour former une pince élastiquement déformable entre une position déployée dans laquelle les branches sont écartées de l'axe médian X-X' et une position repliée dans laquelle les deux branches sont rapprochées de l'axe médian X-X'.

Les deux branches 29A, 29B sont généralement symétriques par rapport à l'axe médian X-X' confondu avec l'axe du conduit, après implantation.

La longueur des branches, mesurée suivant l'axe X-X', est comprise entre 2 et 4 cm et est de préférence égale à 3 cm.

Chaque branche 29A, 29B comporte un tronçon 33A, 33B d'appui sur l'endoprothèse 19. Chaque tronçon d'appui 33A, 33B est constitué d'un segment rectiligne s'étendant généralement suivant une génératrice de l'endoprothèse 19, lorsque l'armature 25 est déployée. Chaque tronçon d'appui 33A, 33B présente une surface extérieure 35A, 35B en appui sur l'endoprothèse 19.

La longueur des tronçons d'appui entre un bord proximal 36A et un bord distal 36B est de 1 à 3 cm et de préférence d'environ 2 cm.

Ces tronçons d'appui 33A, 33B sont prolongés par des tronçons de manoeuvre 37A, 37B convergents l'un vers l'autre jusqu'au point de liaison 31. Les tronçons de manoeuvre sont généralement inclinés par rapport à l'axe médian X-X'.

Les tronçons de manoeuvre 37A, 37B sont généralement courbes et présentent un centre de courbure disposé hors de l'espace délimité entre les deux branches. Ainsi, les tronçons 37A, 37B sont bombés vers l'intérieur de la pince.

L'obturateur 27 est constitué d'une poche souple 39 présentant une ouverture distale 41 généralement circulaire d'axe X-X, lorsque la poche 39 est gonflée.

La poche 39 présente une jupe généralement cylindrique 43 prolongée par un fond 45 généralement hémisphérique. Le fond 45 présente un orifice proximal 47 de petit diamètre par rapport à la section de l'ouverture 41.

La poche 39 est réalisée par exemple en polyuréthanne ou en matériel biologique (péricarde bovin).

La hauteur de la jupe 43 est par exemple égale à 4 ou 5 mm et est de préférence compris entre 2 et 5 mm.

La poche 39 est reliée aux tronçons d'appui 33A, 33B par collage ou tout autre moyen adapté suivant la longueur des génératrices de la jupe 43.

Avantageusement, la poche 39 est liée aux deux branches 29A, 29B, de sorte que les deux demi-jupes délimitées de part et d'autre soient de longueurs légèrement différentes.

Enfin, le fond 45 est relié par des fils 49 aux tronçons de manoeuvre 37A, 37B des deux branches de l'armature de porteuse afin d'éviter un retournement de la poche par invagination.

L'endoprothèse 19 est constituée par exemple d'un treillis tubulaire 55 noyé dans un film 56 extensible et étanche aux liquides tel qu'un élastomère. Le treillis 55 est constitué d'acier inoxydable ayant des propriétés de ressort, de sorte que l'endoprothèse 19 est autoexpansible. Une telle endoprothèse est couramment désignée par le terme anglais « stent ».

Comme connu en soi, l'endoprothèse est susceptible de se déformer spontanément d'un état comprimé, dans lequel elle présente un petit diamètre, vers un état dilaté, dans lequel elle présente un diamètre supérieur, cet état dilaté constituant son état de repos.

Dans son état implanté, tel qu'illustré sur la Figure 2, l'endoprothèse 19 s'applique, du fait de son élasticité, contre la surface interne du conduit 13 constituant ainsi une gaine intérieure au conduit 13.

L'endoprothèse 19 présente ainsi une surface intérieure 57 qui délimite un canal 58 de circulation du flux sanguin, d'axe X-X'.

Comme illustré par la Figure 2, la surface intérieure 57 comprend une partie proximale 59 sensiblement cylindrique, une partie médiane 61 creuse, de dimension transversale supérieure aux dimensions transversales de la partie proximale 59, et une partie distale 63, sensiblement cylindrique de section transversale égale à la section transversale de la partie proximale 59.

La partie médiane 61 est délimitée par un tronçon proximal 65 et un tronçon distal 67 de section transversale variable, reliés entre eux par un tronçon intermédiaire 69 de section transversale constante.

Le tronçon 65 est réalisé par une déformation radiale extérieure de l'armature 55. Il présente une section transversale qui augmente distalement. Le tronçon 65 forme une butée proximale 71 destinée à coopérer avec le bord proximal 36A des tronçons d'appui 33A, 33B.

Le tronçon intermédiaire 69 est de section transversale sensiblement constante et supérieure à la section transversale de la partie proximale 59 et de la partie médiane 61. La longueur du tronçon intermédiaire 69, prise suivant l'axe X-X' est sensiblement égale à la longueur des tronçons d'appui 33A, 33B de la valve 17.

Le tronçon distal 67 présente une section transversale qui diminue distalement. Il forme une butée distale 73 destinée à coopérer avec le bord distal 36B des tronçons d'appui 33A, 33B.

Le tronçon proximal 65 et le tronçon distal 67 sont par ailleurs revêtus d'un matériau radio-opaque facilitant leur détection par rayon X.

Comme illustré par la Figure 1, les moyens 21 d'implantation et d'extraction comprennent un cathéter 81 de section transversale intérieure inférieure à la section transversale de la valve 17 dans son état déployé et de section extérieure inférieure à la section intérieure de l'endoprothèse 19.

Les moyens 21 d'implantation et d'extraction comprennent également un outil 83 de traction et de poussée, munie d'une mâchoire 85 à son extrémité distale.

Avant d'implanter le nécessaire 11 selon l'invention dans l'artère 13, le chirurgien introduit de manière connue l'endoprothèse 19 dans son état comprimé jusqu'à sa position d'implantation dans le conduit 13. Puis, l'endoprothèse 19 est placée dans son état dilaté de sorte que l'armature 25 se plaque contre le conduit 13.

Ensuite, la mâchoire 85 de l'outil de traction 83 saisit l'extrémité 31 de la valve 17. La valve 17 est alors insérée dans le cathéter 81 par traction sur l'outil 83.

La valve 17 présente alors un état rétracté. Le cathéter 81 est introduit dans le conduit 13. Il est amené jusque dans le canal intérieur 58 de l'endoprothèse 21 jusqu'à une position située sensiblement en regard de la partie proximale 59.

Les tronçons d'appui 33A, 33B de la valve 17 sont extraits du cathéter et la valve 17 se déploie jusqu'à son état déployé d'implantation. La valve 17 est alors poussée distalement de sorte que le bord distal 26B des tronçons d'appui pénètre dans la partie médiane creuse 61 délimitée dans la surface intérieure 50 de l'endoprothèse 21.

Lorsque le bord distal 26B de chaque tronçon d'appui 33A, 33B atteint le tronçon distal 67, il bute contre la butée distale 73, ce qui empêche son déplacement distal au-delà de cette butée 73.

Par ailleurs, dans cette position, le bord proximal 26A de chaque tronçon d'appui 33A, 33B est calé contre la butée proximale 71, ce qui empêche son déplacement proximal au-delà de cette butée.

La valve 17 est donc solidement fixée dans le canal intérieur 50 de l'endoprothèse 21.

Par ailleurs, sa position relative par rapport à l'endoprothèse 21 est déterminée de manière précise par les dimensions de la partie médiane creuse 61.

Une telle valve prothétique 17 implantée fonctionne de la manière suivante. A l'issue d'une expulsion du ventricule droit, lorsque celui-ci augmente de volume, le flux sanguin est aspiré dans le conduit 13 de l'extrémité 15B vers l'extrémité 15A. Le sang emplit alors la poche 39, laquelle s'applique sur l'endoprothèse 14, comme illustré sur la Figure 2, obturant ainsi de manière sensiblement étanche le conduit organique 13.

Lors de la circulation du sang, l'orifice 47 permet un écoulement constant d'un faible flux sanguin au travers de la poche 39, évitant la formation d'un caillot au fond de la poche 39 du fait d'une éventuelle stagnation de sang.

En revanche, lors d'une contraction du ventricule droit, le sang circule de l'extrémité 15A vers l'extrémité 15B. La poche 39 est alors sollicitée extérieurement depuis le fond 45 provoquant un aplatissement de cette poche. Ainsi, le flux sanguin est libre de circuler dans le conduit 13 de part et d'autre de la poche 39.

Après implantation d'une telle prothèse, la paroi du conduit organique 13 adhère progressivement avec l'endoprothèse 19. En revanche, l'endoprothèse 19 constitue une gaine formant écran entre la valve prothétique 17 et la paroi du conduit organique 13 évitant ainsi une agglomération du conduit organique 13 et de la valve prothétique 17. Ainsi, le retrait de la valve prothétique 17 est possible.

En particulier, la valve prothétique 17 étant équipée de moyens de compression centripète, celle-ci peut être ramenée dans son état comprimé et évacuée de manière transluminale.

Plus précisément, pour le retrait de la valve prothétique 17, le cathéter 81 est introduit au travers de l'oreillette droite et du ventricule droit et est disposé en regard de l'extrémité 31 de l'armature porteuse en forme de pince.

L'outil de traction 83 est acheminé au travers du cathéter 81. La mâchoire 85 saisit alors l'extrémité 31 de la pince. Alors que l'extrémité ouverte, notée 87, du cathéter est en contact avec les tronçons de manoeuvre 37A, 37B, l'armature porteuse est progressivement tirée à l'intérieur du cathéter 81. Par effet de came, les deux bras 29A, 29B sont resserrés l'un vers l'autre et la valve prothétique est progressivement amenée dans son état resserré et introduite dans le cathéter 81. Le cathéter 81 renfermant la valve prothétique est alors extrait du corps humain.

Un nouveau cathéter contenant une nouvelle valve prothétique est alors introduit dans le corps humain et la valve est larguée en procédant aux opérations exposées précédemment, en sens inverse.

Dans ce deuxième nécessaire 88 selon l'invention, représenté sur la Figure 3, le tronçon intermédiaire 69 présente une section transversale sensiblement égale à la section transversale des parties proximales 59 et distales 63 de la surface intérieure 57.

Les butées proximales 71 et 73 sont formées dans la surface 67 sur des étranglements annulaires proximal et distal 89 et 90 qui font saillie radialement à l'intérieur du canal 58.

Le fonctionnement de ce nécessaire 88 est par ailleurs sensiblement analogue à celui du nécessaire de la Figure 2.

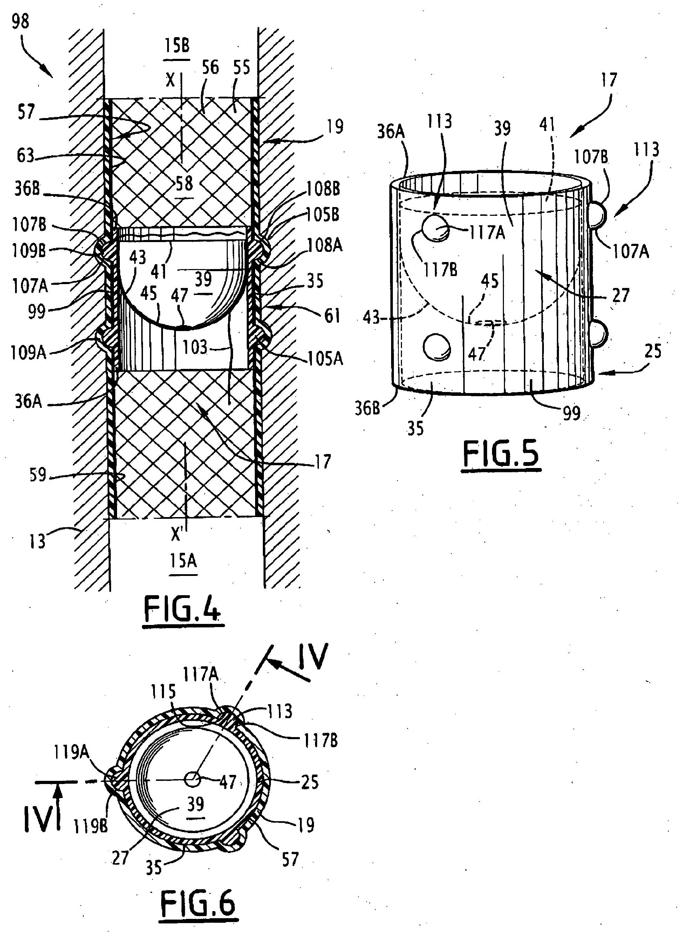

Dans le troisième nécessaire 98 selon l'invention, illustré par la Figure 4, l'armature de la valve 17 est formée par un treillis métallique tubulaire élastique 99. La poche 39 est liée suivant la périphérie ouverte en deux ou trois points sur le treillis tubulaire 99.

La valve prothétique 17 comporte en outre un brin de constriction 103 engagé à demeure dans les différentes boucles délimitées par le treillis 99 suivant une circonférence de celui-ci. Le brin 103 est refermé en boucle. Il est suffisamment long pour permettre l'expansion de la valve 17. Ce brin 103 forme des moyens de compression centripète. La traction sur ce brin 103, par exemple à l'aide d'une pince, provoque une constriction du treillis 99 permettant un retrait de la valve prothétique 17 après engagement de celle-ci dans un cathéter 81.

Par ailleurs, le treillis 99 présente sur sa surface radiale extérieure deux bourrelets annulaires 105A et 105B espacés axialement le long de l'axe X-X'.

Chaque bourrelet 105A, 105B s'étend dans un plan transversal le long de la périphérie de la surface extérieure du treillis 99. Chaque bourrelet 105A, 105B délimite une surface extérieure qui présente un tronçon 107A de section transversale croissante distalement le long de l'axe X-X' et un tronçon 107B de section transversale décroissante distalement le long de l'axe X-X'. Les tronçons 107A, 107B forment respectivement des butées proximale et distale de blocage du déplacement axial de la valve 17.

Par ailleurs, l'endoprothèse 21 comprend deux gorges annulaires 109A, 109B de formes sensiblement complémentaires aux bourrelets annulaires 105A, 105B. Chaque gorge 109A, 109B forme deux fronts 108A et 108B de section transversale variable le long de l'axe X-X' dans la surface intérieure 57 de l'endoprothèse 21.

Lorsque la valve 17 est implantée dans l'endoprothèse 21, les bourrelets annulaires 105A, 105B sont insérés dans les gorges 109A, 109B correspondantes. Les butées 107A et 107B coopérent axialement avec les fronts 108A et 108B correspondants pour empêcher le déplacement axial de la valve 17 par rapport à l'endoprothèse 21 dans deux sens opposés le long de l'axe X-X'.

Dans la variante représentée sur les Figures 5 et 6, la surface exterieure 35 de la valve 17 comprend une pluralité de pions de blocage 113 qui font saillie radialement vers l'extérieur. Dans cet exemple, les pions de blocage 113 sont disposés dans deux plans transversaux espacés axialement. Chaque plan transversal comprend trois pions de blocage 113 répartis angulairement autour de l'axe X-X'.

La surface intérieure 50 de l'endoprothèse 21 délimite des enfoncements 115 de formes correspondantes aux pions de blocage 113. Comme illustré par la Figure 6, chaque pion de blocage 113 délimite deux surfaces de butée angulaire 117A et 117B qui coopèrent avec deux fronts 119A, 119B de butée angulaire correspondants délimités dans les enfoncements 115.

Ainsi, lorsque la valve 17 est implantée dans l'endoprothèse 21 et que les pions 113 sont insérés dans les enfoncements 115 correspondants, la valve 17 est bloquée axialement en translation par rapport à l'endoprothèse 21, et bloquée angulairement autour de l'axe X-X' par rapport à l'endoprothèse 21.

Dans une variante non représentée, les pions 113 sont insérés directement dans les orifices ménagés entre les fils d'armature du treillis 99. L'endoprothèse 19 présente alors une section sensiblement constante le long de l'axe X-X'.

Grâce à l'invention qui vient d'être décrite, il est donc possible de disposer d'un nécessaire d'implantation qui permet de fixer de manière précise, robuste et amovible une valve prothétique 17 dans une endoprothèse tubulaire 21 par des moyens simples et peu coûteux.

## Revendications

1. Nécessaire (11 ; 88 ; 98), destiné à être implanté dans un conduit (13) de circulation du sang, du type comprenant :
- une endoprothèse tubulaire (19) présentant une surface intérieure (57) délimitant un canal (58) d'axe longitudinal (X-X') ; et
- une valve prothétique (17) destinée à être implantée dans le canal (58), la valve (17) comprenant :
• une armature porteuse (25) présentant une surface extérieure (35A, 35B ; 35) destinée à être appliquée contre la surface intérieure (57) de l'endoprothèse (19), l'armature (25) étant déformable radialement depuis une position repliée de mise en place jusqu'à une position déployée d'implantation ;
• un obturateur souple (27), lié à l'armature (25) et déformable entre une position d'obstruction dans laquelle il est étendu transversalement et une position de libération dans laquelle il est contracté transversalement sous l'action du flux circulant dans le canal (58) ;
**caractérisé en ce que** la surface intérieure (57) présente deux tronçons (65, 67) de section transversale variable le long de l'axe longitudinal (X-X'), les tronçons (65, 67) formant respectivement une butée proximale (71) et une butée distale (73) pour bloquer le déplacement axial de la surface extérieure (35A, 35B ; 35) le long de la surface intérieure (57) suivant deux sens opposés, les deux tronçons (65, 67) étant reliés entre eux par un tronçon d'appui (69) en retrait radialement, le tronçon d'appui (69) présentant une longueur sensiblement égale à la longueur de la surface extérieure (35A, 35B) prévue sur l'armature porteuse (25) et délimitant, avec les tronçons (65, 67) de section transversale variable, un logement de réception l'armature porteuse (25).

2. Nécessaire (88) selon la revendication 1, **caractérisé en ce que** les deux tronçons de section transversale variable sont délimités par deux étranglements annulaires (89, 90) qui font saillie dans le canal (58).

3. Nécessaire (11) selon la revendication 1 ou 2, **caractérisé en ce que** les tronçons (65, 67 ; 107A, 107B ; 109A, 109B) de section transversale variable sont radio-opaques.

4. Endoprothèse tubulaire (19), du type présentant une surface intérieure (57) délimitant un canal (58) d'axe longitudinal (X-X'), dans laquelle la surface intérieure (57) présente deux tronçons (65, 67) de section transversale variable le long de l'axe longitudinal (X-X'), les tronçons (65, 67) formant respectivement une butée proximale (71) et une butée distale (73) pour bloquer le déplacement axial suivant deux sens opposés d'une surface extérieure (35A, 35B) prévue sur une valve prothétique (17) destinée à être implantée sur la surface intérieure (57), **caractérisée en ce que** les deux tronçons (65, 67) sont reliés entre eux par un tronçon d'appui (69) en retrait radialement, le tronçon d'appui (69) présentant une longueur sensiblement égale à la longueur de la surface extérieure (35A, 35B) prévue sur une armature porteuse (25) destinée à être appliquée contre la surface intérieure (57) de l'endoprothèse (19), et délimitant, avec les tronçons (65, 67) de section transversale variable, un logement de réception d'une telle armature porteuse (25).

5. Valve prothétique (17) destinée à être implantée dans un canal (58) d'une endoprothèse tubulaire comprenant :
• une armature porteuse (25) présentant une surface extérieure (35A, 35B ; 35) destinée à être appliquée contre la surface intérieure (57) de l'endoprothèse (19), l'armature (25) étant déformable radialement depuis une position repliée de mise en place jusqu'à une position déployée d'implantation ;
• un obturateur souple (27), lié à l'armature (25) et déformable entre une position d'obstruction dans laquelle il est étendu transversalement et une position de libération dans laquelle il est contracté transversalement sous l'action du flux circulant dans le canal (58) ;
**caractérisée en ce que** la surface intérieure (57) présente deux tronçons (65, 67) de section transversale variable le long de l'axe longitudinal (X-X'), les tronçons (65, 67) formant respectivement une butée proximale (71) et une butée distale (73) pour bloquer le déplacement axial de la valve par rapport à l'endoprothèse tubulaire, les deux tronçons (65, 67) étant reliés entre eux par un tronçon d'appui (69) en retrait radialement, le tronçon d'appui (69) présentant une longueur sensiblement égale à la longueur de la surface extérieure (35A, 35B) prévue sur l'armature porteuse (25) et délimitant, avec les tronçons (65, 67) de section transversale variable, un logement de réception l'armature porteuse (25).

## Patentansprüche

1. Vorrichtung (11; 88; 98), die dazu bestimmt ist in eine Blutkreislauf-Ader (13) implantiert zu werden, des Typs aufweisend:
- eine rohrförmige Endoprothese (19), die eine Innenfläche (57) hat, die einen Kanal (58) mit einer Längsachse (X-X') begrenzt, und
- ein prothetisches Ventil (17), das dazu bestimmt ist, in dem Kanal (58) implantiert zu sein, wobei das Ventil (17) aufweist:
• ein Träger-Gestell (25), das eine Außenfläche (35A, 35B; 35) aufweist, die dazu bestimmt ist um gegen die Innenfläche (57) der Endoprothese (19) angesetzt zu werden, wobei das Gestell (25) radial deformierbar ist von einer Platzier-Zusammenfaltposition aus bis zu einer Implantier-Entfaltungsposition,
• ein flexibles Verschlussorgan (27), das mit dem Gestell (25) verbunden ist und das deformierbar ist zwischen einer Verschlussposition, in welcher es transversal ausgebreitet ist, und einer Freigabeposition, in welcher es transversal kontrahiert ist, unter Wirkung des zirkulierenden Flusses in dem Kanal (58),
**dadurch gekennzeichnet, dass** die Innenfläche (57) zwei Abschnitte (65, 67) wechselnden Querschnitts entlang der Längsachse (X-X') hat, wobei die Abschnitte (65, 67) jeweils einen proximalen Anschlag (71) und einen distalen Anschlag (73) haben zum Blockieren der axialen Verlagerung der Außenfläche (35A, 35B, 35) entlang der Innenfläche (57) in zwei entgegengesetzte Richtungen, wobei die beiden Abschnitte (65, 67) unter radialer Einrückung miteinander verbunden sind durch einen Anlageabschnitt (69), wobei der Anlageabschnitt (69) eine Länge aufweist, die im Wesentlichen gleich der Länge der Außenfläche (35A, 35B) ist, die auf dem Trägerstell (25) vorgesehen ist, und, mit den Abschnitten (65, 67) wechselnden Querschnitts, einen Raum zur Aufnahme des Träger-Gestells (25) begrenzt.

2. Vorrichtung (88) gemäß Anspruch 1, **dadurch gekennzeichnet, dass** die beiden Abschnitte wechselnden Querschnitts begrenzt sind durch zwei Ringeinschnürungen (89, 90), die in den Kanal (58) vorstehen.

3. Vorrichtung (11) gemäß Anspruch 1 oder 2, **dadurch gekennzeichnet, dass** die Abschnitte (65, 67; 107A, 107B; 109A, 109B) wechselnden Querschnitts radioopak sind.

4. Rohrförmige Endoprothese (19) des Typs aufweisend eine Innenfläche (57), die einen Kanal (58) mit einer Längsachse (X-X') begrenzt, wobei die Innenfläche (57) zwei Abschnitte (65, 67) wechselnden Querschnittes entlang der Längsachse (X-X') hat, wobei die Abschnitte (65, 67) jeweilig einen proximalen Anschlag (71) und einen distalen Anschlag (73) bilden zum Blockieren der axialen Verlagerung in zwei entgegengesetzte Richtungen einer Außenfläche (35A, 35B), die auf einem prothetischen Ventil (17) vorgesehen ist, das dazu bestimmt ist auf die Innenfläche (57) implantiert zu werden, **dadurch gekennzeichnet, dass** die beiden Abschnitte (65, 67) unter radialer Einrückung miteinander verbunden sind durch einen Anlageabschnitt (69), wobei der Anlageabschnitt (69) eine Länge hat, die im Wesentlichen gleich der Länge der Außenfläche (35A, 35B) ist, die auf einem Träger-Gestell (25) vorgesehen ist, das dazu bestimmt ist, gegen die Innenfläche (57) der Endoprothese (19) angesetzt zu werden, und, mit den Abschnitten (65, 67) wechselnden Querschnitts, einen Raum zur Aufnahme eines solchen Träger-Gestells (25) begrenzt.

5. Prothetisches Ventil (17), das dazu bestimmt ist, in einen Kanal (58) einer rohrförmigen Endoprothese implantiert zu werden, aufweisend:
• ein Träger-Gestell (25), das eine Außenfläche (35A, 35B; 35) hat, die dazu bestimmt ist, gegen die Innenfläche (57) der Endoprothese (19) angesetzt zu werden, wobei das Gestell (25) radial deformierbar ist von einer Platzier-Zusammenfaltposition aus bis zu einer Implantier-Entfaltungsposition,
• ein flexibles Verschlussorgan (27), das mit dem Gestell (25) verbunden ist und deformierbar ist zwischen einer Verschlussposition, in welcher es transversal ausgebreitet ist, und einer Freigabeposition, in welcher es transversal kontrahiert ist, unter der Wirkung des zirkulierenden Flusses in dem Kanal (58),
**dadurch gekennzeichnet, dass** die Innenfläche (57) zwei Abschnitte (65, 67) wechselnden Querschnitts entlang der Längsachse (X-X') hat, wobei die Abschnitte (65, 67) jeweils einen proximalen Anschlag (71) und einen distalen Anschlag (73) bilden zum Blockieren der axialen Verlagerung des Ventils bezüglich der rohrförmigen Endoprothese, wobei die beiden Abschnitte (65, 67) unter radialer Einrückung miteinander verbunden sind durch einen Anlageabschnitt (69), wobei der Anlageabschnitt (69) eine Länge hat, die im Wesentlichen gleich ist der Länge der Außenfläche (35A, 35B), die auf dem Träger-Gestell (25) vorgesehen ist, und, mit den Abschnitten (65, 67) wechselnden Querschnitts, einen Raum zur Aufnahme des Träger-Gestells (25) begrenzt.

## Claims

1. Kit (11; 88; 98) which is intended to be implanted in a blood vessel (13), of the type comprising:
- a tubular endoprosthesis (19) which has an inner surface (57) which delimits a channel (58) having a longitudinal axis (X-X'); and
- a prosthetic valve (17) which is intended to be implanted in the channel (58), the valve (17) comprising:
• a carrier reinforcement (25) which has an outer surface (35A, 35B; 35) which is intended to be pressed against the inner surface (57) of the endoprosthesis (19), the reinforcement (25) being able to be deformed radially from a folded position for placement to a deployed position for implantation;
• a flexible shutter (27) which is connected to the reinforcement (25) and which can be deformed between a blocking position in which it is extended transversely and a release position in which it is contracted transversely under the action of the flow moving in the channel (58); **characterised in that** the inner surface (57) has two portions (65, 67) having a variable cross-section along the longitudinal axis (X-X'), the portions (65, 67) forming a proximal stop (71) and a distal stop (73), to block the axial displacement of the outer surface (35A, 35B; 35) along the inner surface (57) in two opposing directions, the two portions (65, 67) being connected each other by a radially recessed support portion (69) having a length substantially equal to the length of the outer surface (35A, 35B) of the reinforcement (25) and delimiting, with the portions (65, 67) having a variable cross-section, a housing for receiving the reinforcement (25).

2. Kit (88) according to claim 1, **characterised in that** the two portions having a variable cross-section are delimited by two annular contractions (89, 90) which protrude in the channel (58).

3. Kit (88) according to claim 1 or 2, **characterised in that** the portions (65, 67; 107A, 107B; 109A, 109B) having a variable cross-section are radio-opaque.

4. Tubular endoprosthesis (19), of the type having an inner surface (57) which delimits a channel (58) having a longitudinal axis (X-X'), wherein the inner surface (57) has at least two portions (65, 67) which have a variable cross-section along the longitudinal axis, the portions (65, 67) forming a proximal stop (71) and a distal stop (73), respectively, to block the axial displacement in two opposing directions of an outer surface (35A, 35B) provided on a prosthetic valve (17) which is intended to be implanted on the inner surface (57), **characterized in that** the two portions (65, 67) are connected each other by a radially recessed support portion (69) having a length substantially equal to the length of the outer surface (35A, 35B) of a carrier reinforcement (25) which is intended to be pressed against the inner surface (57) of the endoprosthesis (19), and delimiting, with the portions (65, 67) having a variable cross-section, a housing for receiving the carrier reinforcement (25).

5. Prosthetic valve (17) which is intended to be implanted in a channel (58) of a tubular endoprosthesis, comprising:
• a carrier reinforcement (25) which has an outer surface (35A, 35B; 35) which is intended to be pressed against the inner surface (57) of the endoprosthesis (19), the reinforcement (25) being able to be radially deformed from a folded position for placement to a deployed position for implantation;
• a flexible shutter (27) which is connected to the reinforcement (25) and which can be deformed between a blocking position in which it is extended transversely and a release position in which it is contracted transversely under the action of the flow moving in the channel (58); **characterised in that** the outer surface (57) has at least two portions (65, 67) having a variable cross-section along the longitudinal axis (X-X'), the portions (65, 67) forming a proximal stop (71) and a distal stop (73), respectively, to block the axial displacement of the valve relative to the tubular endoprosthesis, the two portions (65, 67) being connected each other by a radially recessed support portion (69), the support portion (69) having a length substantially equal to the length of the outer surface (35A, 35B) of the carrier reinforcement (25) and delimiting, with the portions (65, 67) having a variable cross-section, a housing for receiving the carrier reinforcement (25).
